# FASCICULE DE BREVET EUROPEEN

(11) **EP 0 373 078 B1**
(45) Date de publication et mention de la délivrance du brevet: **16.03.1994**
(21) Numéro de dépôt: 89420475.9
(22) Date de dépôt: 30.11.1989
(51) Int. Cl.: A61F 2/46

(54) **Appareil de pose d'une tête fémorale**
Vorrichtung für das Einsetzen eines Femoralkopfes
Femoral head positioning apparatus

(30) Priorité: 07.12.1988 FR 8816501
(43) Date de publication de la demande: 13.06.1990
(73) Titulaire: FABRIQUE D'IMPLANTS ET D'INSTRUMENTS CHIRURGICAUX SOCIETE A RESPONSABILITE LIMITEE, F-43240 Saint Just Malmont (FR)
(72) Inventeur: Cuilleron, Jean, F-42100 Saint Etienne (FR)
(74) Mandataire: Dupuis, François

(56) Documents cités:
- EP-A- 0 133 393
- EP-A- 0 207 873
- FR-A- 2 572 378
- US-A- 1 831 149
- US-A- 3 830 236
- US-A- 4 100 626
- US-A- 4 686 971

## Description

L'invention concerne un appareil de pose d'une tête fémorale sur la partie correspondante d'une prothèse de hanche.

On sait que ce type d'implant comprend généralement une tige introduite dans le canal centromédullaire du fémur et un col destiné à recevoir une tête fémorale sphérique. Dans la plupart des cas, la tête fémorale présente une empreinte en creux de forme complémentaire à celle du col de la tige. L'empreinte de la tête et le col, sont très souvent coniques.

La tête fémorale est positionnée sur le col de la tige par le praticien, au moment de l'intervention chirurgicale, ce qui pose d'importants problèmes.

La forme sphérique des têtes, les rend difficile à manipuler. Cette difficulté est encore accrue par le fait que le chirurgien porte des gants humides et souvent couverts de sang. Il en résulte des risques de glissement et par conséquent, d'échapper la tête qui peut être détérioriée.

Même dans l'hypothèse où le chirurgien n'échappe pas la tête, ce type de manipulation présente d'autres inconvénients tels que :
- risque de détérioration de l'état de surface de la tête sachant que l'acier inoxydable se raye très facilement.
- risque d'introduction dans l'empreinte de la tête d'impuretés diverses susceptibles de rester prisonnières après mise en place de la tête sur le col de la prothèse.
- difficulté de mise en place de la tête sur le col de la prothèse qui est déjà introduite dans le canal médullaire, étant souligné qu'il est nécessaire d'orienter la tête fémorale pour présenter son empreinte en regard du col.

Par ailleurs, on conçoit que de telles manipulations compromettent fortement la stérilisation des têtes, malgré toutes les précautions prises initialement pour assurer une parfaite stérilisation desdites têtes avant leur mise en place.

Pour tenter de remédier à ces inconvénients, on a proposé un appareil pour la pose d'une tête prothétique sous forme d'une pince comprenant un corps formé par deux branches articulées. L'extrémité des branches forme un logement sphérique pour le maintien temporaire de la tête prothétique. Des zones d'extrémités, opposées au logement sphérique, présentent des oeillets qui forment des moyens permettant de libérer la tête prothétique en vue de son positionnement sur une partie complémentaire fémorale. Cet état de la technique peut être illustré par l'enseignement du brevet EP-A-0133393.

Cependant, la solution décrite dans ce document n'est pas satisfaisante. Notamment, la manipulation de la pince nécessite une certaine dextérité. Des risques d'échapper la tête prothétique ne sont pas exclus.

Par le brevet FR 2572378 on connaît un dispositif de bouchage pour l'introduction manuelle du bouchon dans des bouteilles. Ce dispositif comprend un corps dont une partie est agencée pour recevoir de manière temporaire, le bouchon, tandis qu'une autre partie présente des moyens aptes à libérer le bouchon en vue de son positionnement sur la bouteille.

Toutefois, l'enseignement de ce brevet ne s'applique pas à la pose d'une tête fémorale destinée à être engagée sur le col d'une prothèse.

L'invention s'est fixée pour but de remédier à ces inconvénients, de manière simple, sûre, efficace et rationnelle, en mettant en oeuvre un appareil apte à assurer la pose d'une tête fémorale, sur le col d'une prothèse, sans aucun risque de tomber ledit élément lors de son transfert, tout en respectant les conditions de stérilisation.

Pour résoudre un tel problème, il a été conçu et mis au point, un appareil du type de ceux comprenant un corps dont une partie est agencée pour recevoir, de manière temporaire, la tête fémorale tandis qu'une autre partie présente des moyens aptes à libérer ledit élément en vue de son positionnement sur le col.

Selon l'invention, les moyens, aptes à libérer la tête fémorale, sont constitués par un bouton poussoir monté à libre coulissement dans le corps et dont une extrémité est conformée pour coopérer en appui avec ladite tête, ledit bouton étant manoeuvrable en combinaison avec des agencements dudit corps.

Avantageusement, le problème posé de libérer l'élément de prothèse est résolu en ce que les agencements du corps sont constitués par des empreintes périphériques formées à partir des génératrices et d'une colerette d'appui et de maintien.

Un autre problème que se propose de résoudre l'invention est de pouvoir prépositionner la tête fémorale à l'intérieur de l'appareil avant sa mise en place sur le col de la prothèse en faisant office d'emballage pour ladite tête, tout en respectant les contraintes de stérilisation.

Un tel problème est résolu en ce que la partie du corps agencée pour recevoir de manière temporaire la tête fémorale, coopère d'une manière amovible avec un organe obturateur apte à assurer la protection deladite tête.

Il est ainsi possible de conditionner l'ensemble de l'appareil qui fait office, dans ce cas, à la fois d'ancillaire et d'emballage pour la tête fémorale, dans une ou des coques préalablement stérilisées.

Pour résoudre le problème posé d'assurer le maintien de manière temporaire de la tête fémorale, la partie du corps recevant la tête est constitué par un chambrage interne agencé pour assurer le positionement et le maintien temporaire de ladite tête, tout en permettant sa libération sous un effort de pression appliqué sur le bouton poussoir.

Le problème posé du prépositionnement de la tête fémorale dans l'appareil, pour permettre de la présenter vis à vis du col de la prothèse, est résolu en ce que l'organe obturateur présente une partie interne apte à coopérer avec une partie de la tête, en vue d'assurer son centrage dans le chambrage du corps.

L'invention est exposée, ci-après, plus en détail à l'aide des dessins annexés dans lesquels :
La figure 1 est une vue de face avec coupe partielle de l'appareil, avant mise en place d'une tête fémorale.
La figure 2 est une vue de face avec coupe partielle de l'appareil équipé d'un organe obturateur.
La figure 3 et 4 montre l'utilisation de l'appareil.

Afin de rendre plus concret l'objet de l'invention, on le décrit maintenant d'une manière non limitative en se référent aux exemples de réalisation des figures des dessins.

Les différentes figures des dessins montrent l'application de l'appareil pour la pose d'une tête fémorale (T) sur le col (C) d'une tige.

L'appareil, désigné dans son ensemble par (A), comprend un corps (1) dont une partie est apte à recevoir la tête fémorale sphérique (T). Le corps (1) de l'appareil de forme générale cylindrique, présente un chambrage interne (1a) pour le logement de la tête (T).

Ce chambrage (1a) présente au niveau de son ouverture notamment, directement ou d'une manière rapportée, des agencements (1b) conformés pour assurer le maintien temporaire de la tête (T), tout en permettant son éjection sous l'effet d'un effort appliqué sur ladite tête. Par exemple, l'extrémité ouverte du chambrage (1a) peut présenter une partie de rétention profilée, un bossage ou autre.

Axialement au chambrage (1a), le corps (1) est équipé d'un bouton poussoir (2) monté à libre coulissement . L'extrémité de ce bouton (2) déborde dans le chambrage (1a) et présente une tête (2a) apte à prendre appui sur l'élément sphérique de prothèse (T). L'autre extrémité du bouton (2) déborde de l'extérieur du corps (1) pour être manoeuvrable en combinaison avec des agencements dudit corps. Par exemple, une partie du corps (1) présente des empreintes périphériques (1c) formées à partir des génératrices et combinées avec une collerette d'appui (1d) pour, à la fois, saisir d'une seule main, l'appareil et exercer une poussée sur le bouton (2).

Eventuellement, le bouton (2) peut être asservi à un organe élastique de rappel.

Suivant une autre caractéristique importante de l'invention, le corps (1) de l'appareil , au niveau du chambrage (1a), reçoit d'une manière amovible un organe obturateur (3), apte à assurer la protection de la tête (T) logée dans ledit chambrage.

Cet organe (3) peut être constitué par un bouchon vissé sur la partie correspondante du corps, sans pour celà exclure d'autre mode de fixation démontable tel que clipsage, emmanchement à force...

L'alésage du bouchon (3) présente une portée interne (3a) apte à coopérer avec une empreinte en creux (Ta) que présente d'origine la tête sphérique (T). Ces dispositions permettent d'assurer le prépositionnement de la tête (T), à l'intérieur de l'appareil de façon à ce que l'empreinte (Ta) puisse être présentée en regard et en alignement avec le col (C) de la tige.

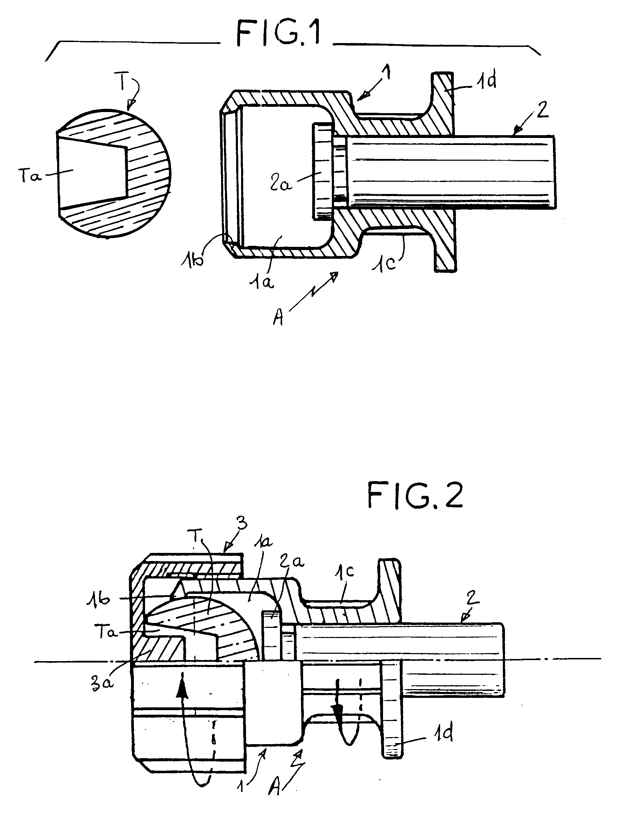

Avantageusement, dans la forme de réalisation illustrée figure 2, l'ensemble de l'appareil (1) équipé du bouchon (3) constitue un emballage pour la tête (T) et un ancillaire de pose de ladite tête (T). L'ensemble de l'appareil peut être en outre conditionné dans une ou plusieurs coques convenable-en outre conditionné dans une ou plusieurs coques convenablement stérilisées. Il en résulte une utilisation particulièrement avantageuse.

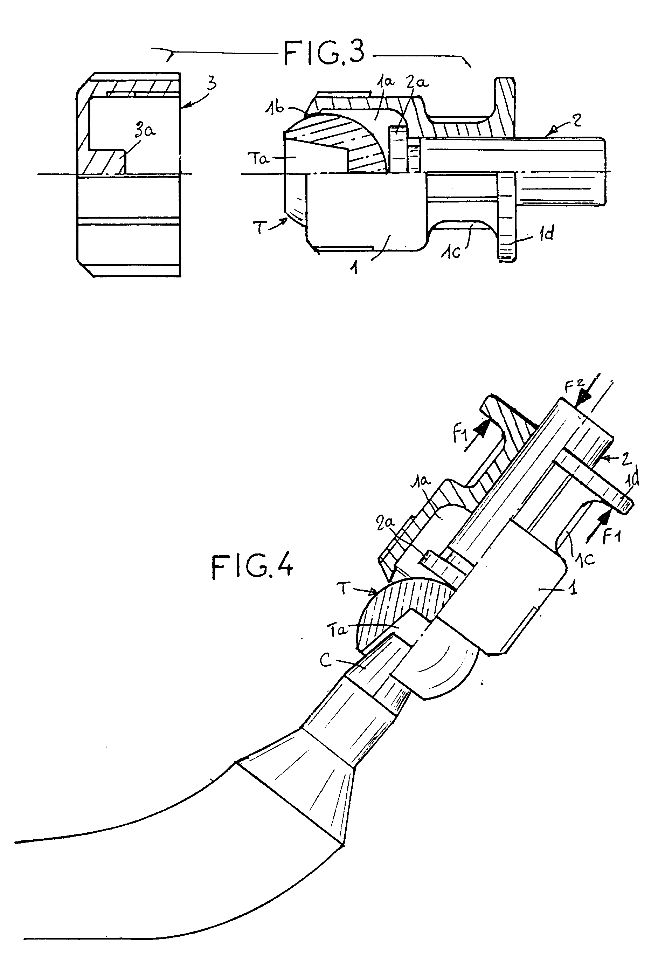

L'opératrice, par exemple, après avoir enlevé l'appareil de la ou des coques de stérilisation (non présenté) enlève le bouchon (3) (figure 3) et présente l'appareil (A) équipé de la tête (T) au chirurgien. Il suffit alors, pour le chirurgien, qui peut parfaitement et facilement saisir l'appareil, de présenter en regard du col (C), la tête (T). L'empreinte interne (Ta) de la tête étant directement en alignement avec le col (C) on exerce sur l'appareil, au niveau du poussoir (2) et de la collerette (1d), des forces F1 et F2, pour provoquer sous l'effet de poussée du bouton (2), l'éjection de la tête (T) qui se positionne presque automatiquement sur la partie complémentaire du col.

Les avantages ressortent bien de la description, en particulier on souligne.
- mise en place sans contact direct avec la tête ce qui n'altère pas sa stérilisation.
- réduction du temps entre la sortie des coques d'emballage stériles et l'implantation sur la prothèse.

L'ensemble de l'appareil peut être exécuté en toute matière et peut constituer un élément jetable ou récupérable.

## Revendications

1. Appareil de pose d'une tête fémorale (T) destinée à être engagée sur le col d'une prothèse, comprenant un corps (1) dont une partie est agencée pour recevoir, de manière temporaire, la tête fémorale (T) tandis qu'une autre partie présente des moyens aptes à libérer ladite tête (T) en vue de son positionnement sur le col (C), caractérisé en ce que les moyens, aptes à libérer la tête fémorale (T), sont constitués par un bouton poussoir (2) monté à libre coulissement dans le corps (1) et dont une extrémité (2a) est conformée pour coopérer en appui avec ladite tête (T), ledit bouton (2) étant manoeuvrable en combinaison avec des agencements dudit corps.

2. Appareil selon la revendication 1, caractérisé en ce que les agencements du corps sont constitués par des empreintes périphériques (1c) formées à partir des génératrices et d'une colerette d'appui et de maintien.

3. Appareil selon la revendication 1, caractérisé en ce que la partie du corps (1) agencée pour recevoir de manière temporaire la tête fémorale (T), coopère d'une manière amovible avec un organe obturateur (3) apte à assurer la protection de ladite tête (T).

4. Appareil selon la revendication 1, caractérisé en ce que la partie du corps (1) recevant la tête (T) est constituée par un chambrage interne (1b) agencé pour assurer le positionement et le maintien temporaire de ladite tête, tout en permettant sa libération sous un effort de pression appliqué sur le bouton poussoir (2).

5. Appareil selon la revendication 2, caractérisé en ce que l'organe obturateur (3) présente une partie interne (3a) apte à coopérer avec une partie (TA) de la tête fémorale (T), en vue d'assurer son centrage dans le chambrage du corps.

## Claims

1. Device for placing a femoral head (T) to be installed on to the neck of a prosthesis, comprising a body (1) part of which is designed to accommodate on a temporary basis the femoral head (T) with another part fitted so as to release the said head (T) in order to position it on the neck (C), characterized in that the means of releasing the femoral head (T) include a push-button (2) which slides freely inside the body (1) with one end (2a) formed so as to support the said head (T), the button (2) being manoeuvrable in conjunction with the body fittings.

2. Device as claimed in Claim 1, characterized in that the body fittings consist of peripheral impressions (1c) formed from generators and of a support and holding collar.

3. Device as claimed in Claim 1, characterized in that the part of the body (1) designed to temporarily accommodate the femoral head (T) interacts with a removable obturator (3) whose function it is to protect the said head (T).

4. Device as claimed in Claim 1, characterized in that the part of the body (1) accommodating the head (T) consists of an internal recess (1b) designed to position and temporarily hold the said head and then release it when pressure is applied to the push-button (2).

5. Device as claimed in Claim 2, characterized in that the obturator (3) includes an internal part (3a) which combines with a part (Ta) of the femoral head (T) to ensure that the head is properly centered in the body recess.

## Patentansprüche

1. Einsetzgerät eines Oberschenkelkopfes (T) zum Aufsetzen auf den Hals einer Prothese, bestehend aus einem Körper (1), von dem ein Teil so ausgebildet ist, daß er zeitweilig den Oberschenkelkopf (T) aufnehmen kann, während ein anderer Teil geeignete Vorrichtungen besitzt, um den besagten Kopf (T) zur Positionierung auf dem Hals (C) freizugeben, dadurch gekennzeichnet, daß die geeigneten Vorrichtungen zur Freigabe des Oberschenkelkopfs (T) aus einem Druckknopf (2) bestehen, der frei in dem Körper (1) gleitet und von dem ein Ende (2a) so ausgebildet ist, daß es auf besagtem Kopf (T) aufliegt, wobei der besagte Knopf (2) in Verbindung mit Ausgestaltungen des genannten Körpers betätigt werden kann.

2. Gerät nach Anspruch 1, dadurch gekennzeichnet, daß die Ausgestaltungen des Körpers (1) aus Randvertiefungen (1c), die aus den Mantellinien gebildet sind, und aus einem Stütz- und Haltekragen bestehen.

3. Gerät nach Anspruch 1, dadurch gekennzeichnet, daß der Teil des Körpers, der für die zeitweilige Aufnahme des Oberschenkelkopfs (T) ausgelegt ist, mit einem abnehmbaren Verschlußorgan (3) zusammenwirkt, das in der Lage ist, für den Schutz des besagten Kopfes (T) zu sorgen.

4. Gerät nach Anspruch 1, dadurch gekennzeichnet, daß der den Kopf (T) aufnehmende Teil des Körpers (1) innen eine Einsenkung (1b) zum Positionieren und zeitweiligen Festhalten des besagten Kopfes besitzt, dabei aber seine Freigabe durch Druckausübung auf den Druckknopf (2) ermöglicht.

5. Gerät nach Anspruch 2, dadurch gekennzeichnet, daß das Verschlußorgan (3) ein inneres Teilstück (3a) aufweist, das dazu geeignet ist, zur sicheren Zentrierung in der Einsenkung des Körpers mit einem Teil (Ta) des Oberschenkelkopfes (T) zusammenzuwirken.
